# EUROPÄISCHE PATENTANMELDUNG

(11) **EP 1 502 581 A1**
(43) Veröffentlichungstag der Anmeldung: **02.02.2005**
(21) Anmeldenummer: 04102782.2
(22) Anmeldetag: 17.06.2004
(51) Int. Cl.: A61K 7/15

(54) **Tensidfreie Rasierhilfszubereitung**

(30) Priorität: 01.08.2003 DE 10336044
(71) Anmelder: Beiersdorf AG, 20253 Hamburg (DE)
(72) Erfinder: Heike, Kerstin, 22529, Hamburg (DE); Treu, Jens, 22844, Norderstedt (DE); Post, Katharina, 20359, Hamburg (DE); Wolter, Kathrin, 22251, Hamburg (DE)

(57) **Zusammenfassung**

Lipidhaltige, dabei emulgatorhaltige oder emulgatorfreie oder aber lipidfreie und emulgatorfreie Rasierhilfszubereitung geeignet für Elektrorasierer enthaltend
0,05 bis 0,5 Gew.% vernetzte Polyacrylate,
1 bis 7 Gew.% Glycerin ,
0,1 bis 1 Gew.% Xanthan-Gummi
und Wasser,
wobei die Zubereitung einer Viskosität von 500 bis 5000 mPas aufweist, gemessen mit dem Haake Viscosimeter 'Viscotester VT02' gemessen bei Raumtemperatur in einem Gefäß mit dem Durchmesser von 42 mm und Ablesen des Meßwertes nach 30 s, dadurch gekennzeichnet das die Zubereitung frei von Tensiden, insbesondere frei von Sarcosinaten ist.

## Beschreibung

Die vorliegende Erfindung betrifft Zubereitungen, die auf bestimmte Rasiererapparate zugeschnitten sind. Die Erfindung umfasst dabei lipidhaltige, beispielsweise als Emulsionen oder Gelcremes vorliegende Zubereitungen und lipidfreie beispielsweise als Gele vorliegende Zubereitungen. Lipidhaltige Zubereitungen können auch frei von Emulgatoren sein und werden dann als Gelcremes bezeichnet. All diesen Zubereitungen ist aber gemein, dass sie frei von Tensiden, insbesondere frei von Sarcosinaten formuliert werden können.

Rasieren dient der mechanischen Entfernung von Haaren von der Haut durch Abschneiden des Barthaares. Dabei gibt es zwei bekannte Verfahren: das Nass- oder das Trockenrasieren. Beim Nassrasieren wird zunächst durch Auftragen einer seifenhaltigen Rasierhilfszubereitung oder eines seifenhaltigen Schaums das Haar weich und flexibel gemacht. Anschließend zu wird das Haar mit einer Klinge abgeschnitten. Dabei gleitet die Klinge über die Haut und reizt diese. Dieser Hautreizung kann man entgegenwirken, indem man Rasierhilfszubereitungen wählt, die besonders leichtes Gleiten der Klinge über die Haut erlauben. Eine andere Möglichkeit ist die trockene Rasur. Bei der trockenen Rasur gleitet keine Klinge direkt über die Haut, sondern ein so genanntes Scherblatt, hinter dem die Klinge verborgen ist. Die Klinge ist elektrisch angetrieben und schneidet durch ihre Bewegungen Haare ab, die durch das Scherblatt hindurch treten. Die Klinge kommt auf diese Weise nicht indirekten Kontakt zur Haut. Verwendet man Zubereitungen, die das Rasierergebnis verbessern sollen, so sollen diese das Haar steif machen, damit es gut durch das Scherblatt hindurch treten kann. Dies würde beispielsweise erreicht durch Alkohol enthaltende Zubereitungen, die dem Haar Feuchtigkeit entziehen und es dadurch besonders hart machen.

Verwendet man einen elektrischen Trockenrasierer mit einer üblichen Rasierhilfszubereitung für die Nassrasur, so erhält man ein unbefriedigendes Ergebnis.

Ein Rasierer, wie er in der europäischen Patentanmeldung WO 98/08659 beschrieben ist stellt eine Zwischenform zwischen Nass- und Trockenenrasierer dar. Er weist wie ein klassischer Trockenrasierer ein Scherblatt auf, zusätzlich kann aber eine Rasierhilfszubereitung dosiert werden, so dass während der Rasur die Haut mit der Rasierhilfszubereitung in Kontakt gebracht wird. Dadurch gleitet der Rasierer besonders gut über die Haut, wenn die Rasierhilfszubereitungen diesem Umstand Rechnung trägt. Derartige Rasierhilfszubereitungen können als Emulsion (lipid - und emulgatorhaltig), Gelcreme (lipidhaltig und emulgatorfrei) oder als Gel (lipid- und emulgatorfrei) vorliegen. Um ein gutes Ergebnis zu erhalten, kommt es u. a. darauf an, dass der Gleitfilm nicht zu dick oder zu zäh ist, und es nicht zu einer Schaumbildung kommt, die den Abstand zwischen Haut und Rasierer vergrößert. Ein weiteres Problem ist das Verkleben des Rasierers selbst nach Beendigung der Rasur. Wird der Rasierer nicht regelmäßig mit Wasser gespült, so bildet sich in der Haarkammer des Rasierers und auf Klingen und Scherblatt ein Depot aus Bartstoppeln und Rasierhilfszubereitung. Außerdem tritt das Problem der Korrosion auf, da die Klinge im Gegensatz zu Trockenrasierern mit Feuchtigkeit in Kontakt kommt und dadurch korrodiert. Könnte eine korrosionsverhindernde Rasierhilfszubereitung bereitgestellt werden, könnte der Anwender beispielsweise die Rasur unter der Dusche durchführen.

Zusammenfassend bedeutet dies, daß die vier Hauptanforderungen (hohe Gleitfähigkeit, kein Verkleben des Scherblattes, keine Korrosion, keine Hautreizung) an ein derartiges Produkt gleichzeitig erfüllt werden müssen, die zur Erfüllung einzelner Anforderungen üblicherweise eingesetzten Hilfsstoffe aber jeweils eine andere Anforderung unerfüllbar machen.

Zusätzlich soll die Rasierhilfszubereitung an die Haut der zu rasierenden Person angepasst werden: eine lipidfreie Rasierhilfszubereitung, die beispielsweise als Gel vorliegen kann, ist besonders für fettige Haut oder für Haut die zu Aknebildung neigt, geeignet. Dabei muss aber sichergestellt werden, daß eine ausreichende Gleitfähigkeit des Rasierers auf der Haut erreicht werden kann, da dies üblicherweise durch den Ölgehalt bewirkt wird.

Eine öl- oder lipidhaltige Rasierhilfszubereitung, die beispielsweise als Emulsion oder Gelcreme vorliegen kann, dient dagegen der Pflege der Haut und ist besonders für trockene Haut geeignet. Übliche für die Trockenrasur verwendete ölhaltige Rasierhilfszubereitungen weisen entweder fettige oder feuchte Haptik auf. Beide Formen sind für die Elektrorasur im Sinne der Erfindung ungeeignet. Erstere schmieren die Haut zwar gut, so daß ein ausreichendes Gleiten des Rasierers auf der Haut erreicht wird, bilden dabei aber eine Schicht auf der Haut, wodurch die verbleibende Haarlänge nach der Rasur erhöht und somit ein schlechtes Rasurergebnis erzielt wird. Dagegen ziehen feuchte ölhaltige Rasierhilfszubereitungen des Standes der Technik schnell in die Haut ein und stellen somit nicht ausreichend dauerhaft die erforderliche Gleitfähigkeit bereit.

Für Mischhaut es ist von Vorteil, wenn zwar ein gewisser ÖI- oder Lipidanteil in der Rasierhilfszubereitung vorhanden ist, diese aber von Emulgatoren frei ist und die beispielsweise als Gelcreme vorliegen kann. Ein Gehalt an Tensiden oder Emulgatoren ist in jedem Fall ungeeignet, da diese Inhaltsstoffe häufig zu Hautreizungen führen.

Die Schrift WO 2002/87519 offenbart Rasierzubereitungen, die Wasser, ein reinigendes oder konditionierndes Agens für Haut oder Haar und mindestens ein wasserlösliches Polymer enthält und das Schubspannungsverhältnis (Normalspannung /Scherspannung) größer als 3 ist, gemessen bei einer Scherrate von 800 s⁻¹. Über Zubereitungen, die keine Tenside enthalten, wird nichts berichtet.

Die Schrift US 2003/0026775 offenbart Rasierzubereitungen, die Wasser, ein schaumbildendes Tensid und ein flüchtiges Aufschäummittel enthält und bestimmte rheologische Eigenschaften aufweist. Über Zubereitungen, die keine Tenside enthalten, wird nichts berichtet.

DE 10057925 offenbart Zubereitungen, insbesondere Rasiergele, welche zur Rasur mittels eines mechanischen Rasiergerätes, insbesondere eines elektrischen Rasiergerätes, geeignet sind und die einen Gehalt an einem oder mehreren N-Acylsarkosinaten, einem oder mehreren Polysacchariden, einer oder mehreren Substanzen, welche in Wasser ein Gelgerüst bilden, welche keine Polysaccharide darstellen und welche vorteilhafterweise grenzflächenaktive Eigenschaften aufweisen, einem oder mehreren Lösungsvermittlern, bevorzugt gewählt aus der Gruppe der polyethoxylierten gesättigten Fett- oder Ölkomponenten und Wasser aufweisen. Über Zubereitungen, die keine Tenside enthalten, wird nichts berichtet.

All diese Zubereitungen sind zwar für die Elekrtro- oder Trockenrasur geeignet, führen aber regelmäßig zumindest bei einigen Anwendern zu Hautreizungen. Davon ausgehend lag der vorliegenden Erfindung die Aufgabe zugrunde, Zubereitungen zu finden, die besonders hautfreundlich sind. Es hat sich für den Fachmann nicht vorhersehbar herausgestellt, dass lipidhaltige, dabei emulgatorhaltige oder emulgatorfreie oder aber lipidfreie und emulgatorfreie Rasierhilfszubereitungen geeignet für Elektrorasierer enthaltend
0,05 bis 0,5 Gew.% vernetzte Polyacrylate,
1 bis 7 Gew.% Glycerin ,
0,1 bis 1 Gew.% Xanthan-Gummi
und Wasser,
wobei die Zubereitung einer Viskosität von 500 bis 5000 mPas aufweist, gemessen mit dem Haake Viscosimeter 'Viscotester VT02' gemessen bei Raumtemperatur in einem Gefäß mit dem Durchmesser von 42 mm und Ablesen des Meßwertes nach 30 s, dadurch gekennzeichnet das die Zubereitung frei von Tensiden, insbesondere frei von Sarcosinaten ist, den Mängeln des Standes der Technik abhelfen.

Erfindungsgemäße Rasierhilfszubereitungen zeichnen sich durch außerordentliche Milde, gute Gleitfähigkeit des Rasierapparates auf der mit Rasierhilfszubereitung benetzten Hautoberfläche und gute pflegende Eigenschaften aus. Dabei fühlen sich solche Zubereitungen auf der Haut frisch und kühlend an und befeuchten die Haut gut. Solche Zubereitungen könne als Gel, lipidhaltiges Gel (Gelcreme) oder als Creme vorliegen. Im Falle eines Gels besteht der Vorteil solcher Zubereitungen insbesondere darin, dass die Gleitfähigkeit des Rasierers auf der Haut erheblich verbessert wird, ohne das zusätzlich Öle eingearbeitet werden müssen. Damit sind diese Zubereitungen besonders für fettige oder zu Akne neigende Haut geeignet. Gleichzeitig können diese Zubereitungen nun tensidfrei bereitgestellt werden. Im Falle eines lipidhaltigen Gels (Gelcreme) besteht der Vorteil solcher Zubereitungen insbesondere darin, dass wenig Lipid, kein Emulgator und kein Tensid eingesetzt werden müssen und die Zubereitung dennoch ausreichendes Gleiten des Rasierers über die Haut ermöglicht. Solche Zubereitung sind insbesondere für so genannte Mischhaut geeignet. Im Falle einer Creme besteht der Vorteil solcher Zubereitungen insbesondere darin, dass die Haut pflegende Lipide und kein Tensid eingesetzt werden können und die Zubereitung damit eine Pflege der Haut während des Rasierens ermöglicht.

Es wurde weiter gefunden, daß es im Falle lipidhaltiger Zubereitungen bevorzugt ist, wenn zusätzlich Polyethylenglycole enthalten sind. Besonders bevorzugt wird/werden das oder die Polyethylenglycole gewählt aus der Gruppe PEG 20, 30, 45 und 90. Ganz besonders bevorzugt ist PEG 45. Durch die Einarbeitung von Polyethylenglycolen lässt sich ein besseres Gleitvermögen des Rasierapparates über die Haut erreichen.

Es ist besonders bevorzugt, wenn der Gehalt an Lipiden und/oder Ölen 1 bis 5 Gew.% beträgt. Dadurch wird eine optimale Pflegeleistung und Sensorik erreicht.

Weiterhin ist es bevorzugt, wenn in solchen Rasierhilfszubereitungen vorliegend als O/W-Emulsion der Gehalt an Emulgatoren 0,5 bis 3% beträgt. Damit lassen sich unterschiedliche Lipidgehalte und Viskositäten stabil formulieren.

Bevorzugt werden die Lipide gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, besonders bevorzugt Ethyl Hexyl Cocoate.

Weiterhin ist es bevorzugt, wenn in solchen Rasierhilfszubereitungen vorliegend als O/W-Emulsion die Emulgatoren gewählt werden aus der Gruppe der Mono-, Di- und Trialkylphosphosäureester und deren Ethoxylate besonders bevorzugt Triceteareth-4 Phopsphat.

Im Falle lipidfreier und emulgatorfreier Rasierhilfszubereitung vorliegend als Gel ist es besonders bevorzugt, wenn zusätzlich ein Gehalt an PEG-40-hydriertem Rizinusfettsäureether von 0,75 bis 2 Gew.% vorliegt.

In all diesen lipidfreien Rasierhilfszubereitungen ist es besonders bevorzugt, wenn zusätzlich ein Gehalt an Zellulosederivaten, bevorzugt Hydroxyethylcellulose in Konzentrationen von 0,1 bis 1 Gew.%, bevorzugt 0,1 bis 0,6 Gew.%, besonders bevorzugt 0,2 Gew.% vorliegt.

Die Erfindung umfasst weiterhin die Verwendung von solchen Rasierhilfszubereitungen als Hilfsmittel bei der Elektrorasur sowie einen Rasierapparat enthaltend solche Rasierhilfszubereitungen.

Das Weglassen eines einzelnen Bestandteile beeinträchtigt die einzigartigen Eigenschaften der Gesamtzusammensetzung. Daher sind alle angegebenen Bestandteile der erfindungsgemäßen Rasierhilfszubereitungen zwangsläufig erforderlich, um die Erfindung auszuführen.

Vorteilhaft ist auch ein Gehalt an Xanthangummi. Xanthangummi (CAS-Nr. 11138-66-2) ist ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von 2×10⁶ bis 24×10⁶ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan ist die Bezeichnung für das erste mikrobielle anionische Heteropolysaccharid. Es wird von Xanthomonas campestris und einigen anderen Species unter aeroben Bedingungen mit einem Molekulargewicht von 2-15 10⁶ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit z. B. 2-Propanol. Xanthan wird anschließend getrocknet und gemahlen. Xanthan ist gekennzeichnet durch die Struktur 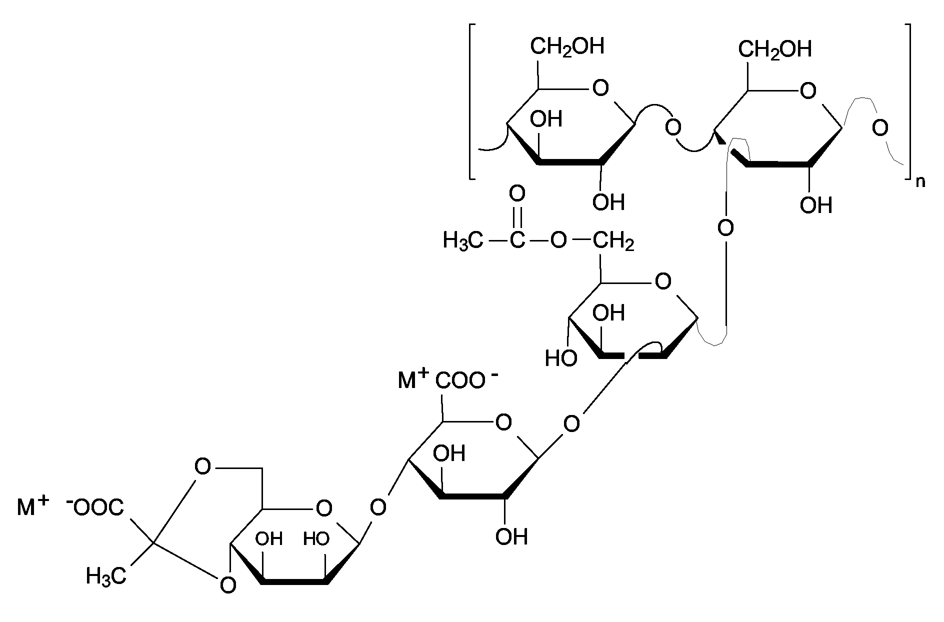 wobei M⁺ Na⁺, K⁺ oder ein halbes Ca²⁺-Äquivalent bedeuten können.

Erfindungsgemäß enthalten die Zubereitungen vorteilhaft 0,01 bis 10 Gew.-%, besonders vorteilhaft 0,01 bis 5 Gew.-%, ganz besonders bevorzugt 0,5 bis 3 Gew.-% an einem oder mehreren Polysacchariden, jeweils bezogen auf das Gesamtgewicht der Zubereitungen.

Vernetzte Polyacrylate im Sinne der Erfindung sind Acrylat-Alkylacrylat-Copolymere, insbesondere solche, die aus der Gruppe der sogenannten Carbomere oder Carbopole (Carbopol® ist eigentlich eine eingetragene Marke der B. F. Goodrich Company) gewählt werden. Insbesondere zeichnen sich das oder die erfindungsgemäß vorteilhaften Acrylat-Alkylacrylat-Copolymere durch die folgende Struktur aus:

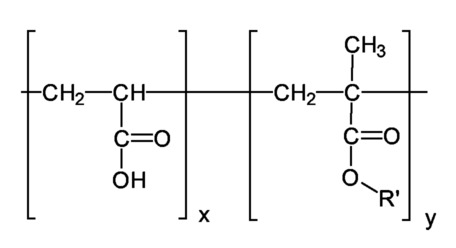

Darin stellen R' einen langkettigen Alkylrest und x und y Zahlen dar, welche den jeweiligen stöchiometrischen Anteil der jeweiligen Comonomere symbolisieren.

Ferner vorteilhaft sind Copolymere aus C₁₀₋₃₀-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Erfindungsgemäß besonders vorteilhaft sind volgende Polyacrylattypen:

| | |
|---|---|
| Carbomer 907 | (Molgew. 450 000), |
| Carbomer 910 | (Molgew. 750 000), |
| Carbomer 941 und Carbomer 951 | (Molgew. 1 250 000), |
| Carbomer 934, | |
| Carbomer 940 und Carbomer 954 | (Molgew. 3 000 000), |
| Carbomer 940 | (Molgew. 4 000 000), |
| Carbomer 980, | (Molgew. 4 000 000), |
| Carbomer 981 | (Molgew. 1 250 000), |
| Carbomer 984 | (Molgew. 3 000 000), |
| (Carbomer 980, 981 und 984 sind Acrylsäure-Polymerisate, die in einem Gemisch aus Ethylacetat und Cyclohexan polymerisiert werden) | |
| Carbomer 974 P | (Molgew. 3 000 000), |
| Carbomer 1342 | (Molgew. 1 300 000). |

Carbomer 1342 weist eine ähnliche Zusammensetzung auf wie die Pemulen-Typen. Pemulen ist die Handelsbezeichnung für ein Copolymeres aus C₁₀₋₃₀-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit (CTFA-Bezeichnung: Acrylates/C₁₀₋₃₀ Alkyl Acrylate Crosspolymer). Bei den Pemulenen handelt es sich um hochmolekulare Polymere mit einem hohen hydrophilen und zugleich mit einem geringen Gehalt an lipophilen Anteilen.

Carbomer 1342 ist die erfindungsgemäß bevorzugte Substanz, welche in Wasser ein Gelgerüst bildet.

Der Wassergehalt der erfindungsgemäßen Zubereitungen beträgt in der Regel zwischen 70 und 98 Gew.-%, bezogen auf das Gesamtgewicht der Zubereitungen, insbesondere zwischen 85 und 95 Gew.-%.

Zellulosederivate im Sinne der Erfindung sind Celluloseether, beispielsweise Methyl- oder Hydroxyethylcellulosen. Sie zeichnen sich durch die folgende Strukturformel aus

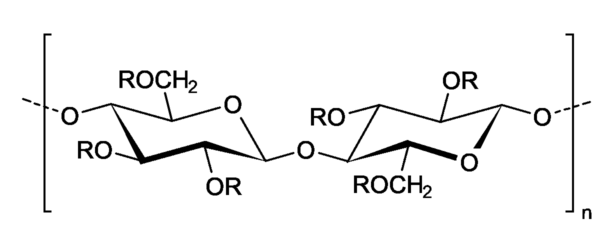

in der R ein Wasserstoff, Alkyl- oder eine Hydroxyalkylgruppe darstellen kann.

Insbesondere vorteilhaft im Sinne der vorliegenden Erfindung sind die Cellulosemischether, die nebeneinander Methyl-2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder CH₂-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Ganz besonders bevorzugt ist Hydroxyethylcellulose.

Vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen ei ner Kettenlänge von 3 bis 30 C-Atomen. Solche Esteröle können dann vorteilhaft gewählt werden aus der Gruppe Octylpalmitat, Ethylhexyl Cocoate Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester.

Ferner kann die Ölphase vorteilhaft gewählt werden aus der Gruppe der Dialkylether und Dialkylcarbonate.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene und hydrierte Polyisobutene die bevorzugten Substanzen.

Die unpolaren Ölkomponenten können vorteilhaft in einer Konzentration von bis zu 60 Gewichts-% bezogen auf das Gesamtgewicht der Lipidphase in den erfindungsgemäßen Zubereitungen enthalten sein.

Die erfindungsgemäßen Zubereitungen, welche in Form einer Emulsion vorliegen, enthalten einen oder mehrere Emulgatoren. Diese Emulgatoren können vorteilhaft gewählt werden aus der Gruppe der nichtionischen, anionischen, kationischen oder amphoteren Emulgatoren.

Unter den nichtionischen Emulgatoren befinden sich
a) Partialfettsäureester und Fettsäureester mehrwertiger Alkohole und deren ethoxylierte Derivate (z. B. Glycerylmonostearate, Sorbitanstearate, Glycerylstearylcitrate, Sucrosestearate)
b) ethoxylierte Fettalkohole und Fettsäuren
c) ethoxilierte Fettamine, Fettsäureamide, Fettsäurealkanolamide
d) Alkylphenolpolyglycolether (z.B. Triton X)
e) Zuckerderivate (Ester und/oder Ether von Glucose, Saccharose und anderen Zuckern; z.B. Alkylpolyglycoside wie Polyglyceryl-3-Methylglucose-Distearat, Methylglucosesesquistearat )

Unter den anionischen Emulgatoren befinden sich
a) Seifen (z. B. Natriumstearat)
b) Fettalkoholsulfate
c) Mono-, Di- und Trialkylphosphosäureester und deren Ethoxylate wie z. B. Triceteareth-4 Phopsphat.

Unter den kationischen Emulgatoren befinden sich
a) quaternäre Ammoniumverbindungen mit einem langkettigen aliphatischen Rest z.B. Distearyldimonium Chloride

Unter den amphoteren Emulgatoren befinden sich
a) Alkylamininoalkancarbonsäuren
b) Betaine, Sulfobetaine
c) Imidazolinderivate

Weiterhin gibt es natürlich vorkommende Emulgatoren, zu denen Bienenwachs, Wollwachs, Lecithin und Sterole gehören.

O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel

   R-O-(-CH₂-CH₂-O-)ₙ-R',
- der Fettsäureethoxylate der allgemeinen Formel

   R-COO-(-CH₂-CH₂-O-)ₙ -H,
- der veretherten Fettsäureethoxylate der allgemeinen Formel

   R-COO-(-CH₂-CH₂-O-)ₙ-R',
- der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

   R-O-(-CH₂-CH₂-O-)ₙ-CH₂-COOH

   nd n eine Zahl von 5 bis 30 darstellen,
- der Polyoxyethylensorbitolfettsäureester,
- der Alkylethersulfate der allgemeinen Formel

   R-O-(-CH₂-CH₂-O-)ₙ-SO₃-H
- der Fettalkoholpropoxylate der allgemeinen Formel

   R-O-(-CH₂-CH(CH₃)-O-)ₙ-H,
- der Polypropylenglycolether der allgemeinen Formel

   R-O-(-CH₂-CH(CH₃)-O-)ₙ-R',
- der propoxylierten Wollwachsalkohole,
- der veretherten Fettsäurepropoxylate

   R-COO-(-CH₂-CH(CH₃)-O-)ₙ-R',
- der veresterten Fettsäurepropoxylate der allgemeinen Formel

   R-COO-(-CH₂-CH(CH₃)-O-)ₙ-C(O)-R',
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)O-)ₙ-CH₂-COOH
- der Alkylethersulfate bzw. die diesen Sulfaten zugrundeliegenden Säuren der allgemeinen Formel

   R-O-(-CH₂-CH(CH₃)-O-)ₙ-SO₃-H
- der Fettalkoholethoxylate/propoxylate der allgemeinen Formel

   R-O-Xₙ-Yₘ-H,
- der Polypropylenglycolether der allgemeinen Formel

   R-O-Xₙ-Yₘ-R',
- der veretherten Fettsäurepropoxylate der allgemeinen Formel

   R-COO-Xₙ-Yₘ-R',
- der Fettsäureethoxylate/propoxylate der allgemeinen Formel

   R-COO-Xₙ-Yₘ-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20).

Des weiteren ist es erfindungsgemäß günstig PEG-Derivate einzusetzten um das Gleitvermögen des Rasiers auf der Haut positiv zu beeinflussen . Als besonders geeignet im Sinne der Erfindung zeigen sich da vor allem langkettige PEG-Derivate wie PEG 20, 30, 45 und 90.

Die Viskosität im Sinne der Erfindung wird gemessen mit dem Haake Viscosimeter 'Viscotester VT02' bei Raumtemperatur in einem Gefäß mit dem Durchmesser von 42 mm und Ablesen des Meßwertes nach 30 s.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, pH-regulierende Stoffe, organische Lösungsmittel oder Silikonderivate.

Es ist erfindungsgemäß ferner gegebenenfalls vorteilhaft, den Zubereitungen Komplexbildner zuzufügen.

Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden.

Komplexbildner, insbesondere Chelatoren, bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. -lon zu Ringen geschlossen. Die Zahl der gebundenen Liganden hängt von der Koordinationszahl des zentralen Metalls ab. Voraussetzung für die Chelatbildung ist, daß die mit dem Metall reagierende Verbindung zwei oder mehr Atomgruppierungen enthält, die als Elektronendonatoren wirken.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen) und Tetrasodium Iminodisuccinate.

Der oder die Komplexbildner sind erfindungsgemäß vorteilhaft in kosmetischen oder dermatologischen Zubereitungen bevorzugt zu 0,01 Gew.-% bis 10 Gew.-%, bevorzugt zu 0,05 Gew.-% bis 5 Gew.-%, insbesondere bevorzugt zu 0,1 - 2,0 Gew.-%, bezogen auf das Gesamtgewicht der Zubereitungen, enthalten.

Es ist bei all diesem im Einzelfalle möglich, daß die vorgenannten Konzentrationsangaben leicht über- oder unterschritten werden und dennoch erfindungsgemäße Rasierhilfszubereitungen erhalten werden. Dies kommt angesichts der breit streuenden Vielfalt an geeigneten Komponenten derartiger Rasierhilfszubereitungen für den Fachmann nicht unerwartet, so daß er weiß, daß bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

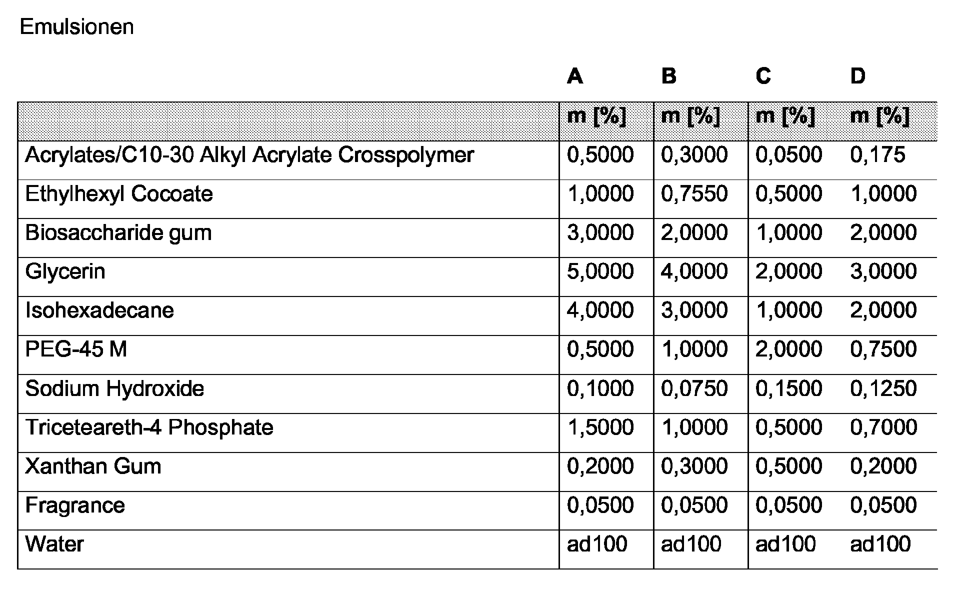

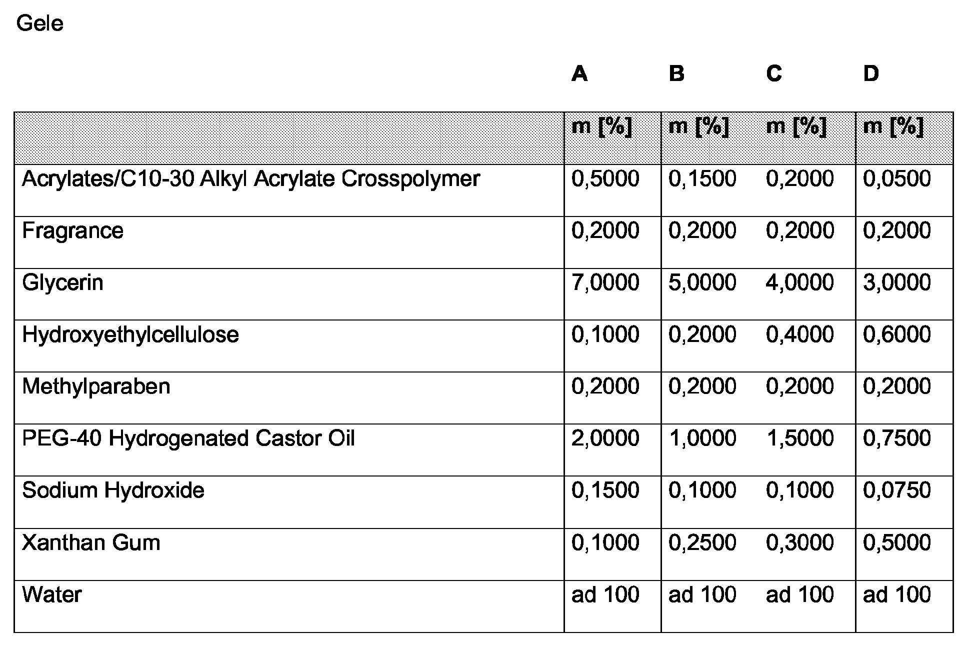

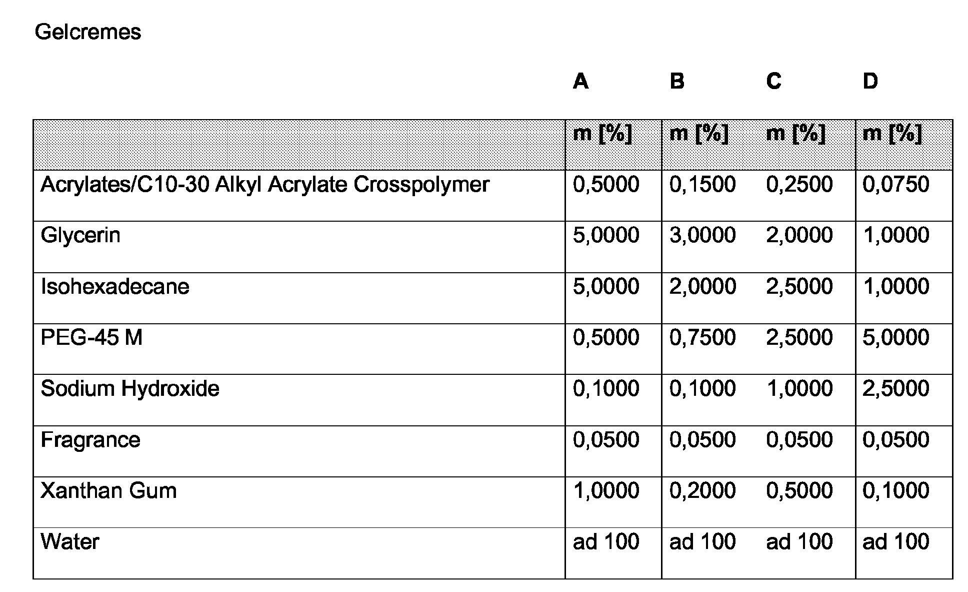

## Patentansprüche

1. Lipidhaltige, dabei emulgatorhaltige oder emulgatorfreie oder aber lipidfreie und emulgatorfreie Rasierhilfszubereitung geeignet für Elektrorasierer enthaltend
0,05 bis 0,5 Gew.% vernetzte Polyacrylate,
1 bis 7 Gew.% Glycerin ,
0,1 bis 1 Gew.% Xanthan-Gummi
und Wasser,
wobei die Zubereitung einer Viskosität von 500 bis 5000 mPas aufweist, gemessen mit dem Haake Viscosimeter 'Viscotester VT02' gemessen bei Raumtemperatur in einem Gefäß mit dem Durchmesser von 42 mm und Ablesen des Meßwertes nach 30 s, **dadurch gekennzeichnet** das die Zubereitung frei von Tensiden, insbesondere frei von Sarcosinaten ist.

2. Lipidhaltige Rasierhilfszubereitung nach Patentanspruch 1 **dadurch gekennzeichnet, daß** zusätzlich Polyethylenglycole enthalten sind.

3. Lipidhaltige Rasierhilfszubereitung nach einem der vorangehenden Ansprüche **dadurch gekennzeichnet, daß** das oder die Polyethylenglycole gewählt werden aus der Gruppe PEG 20, 30, 45 und 90.

4. Lipidhaltige Rasierhilfszubereitung nach einem der vorangehenden Ansprüche **dadurch gekennzeichnet, daß** sie als Polyethylenglycol PEG-45 enthalten.

5. Lipidhaltige Rasierhilfszubereitung nach einem der vorangehenden Ansprüche **dadurch gekennzeichnet, daß** der Gehalt an Lipiden und/oder Ölen 1 bis 5 Gew.% beträgt.

6. Lipidhaltige Rasierhilfszubereitung nach einem der vorangehenden Ansprüche vorliegend als O/W-Emulsion **dadurch gekennzeichnet, daß** der Gehalt an Emulgatoren 0,5 bis 3% beträgt.

7. Lipidhaltige Rasierhilfszubereitung nach einem der vorangehenden Ansprüche **dadurch gekennzeichnet, daß** die Lipide gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, besonders bevorzugt Ethyl Hexyl Cocoate.

8. Lipidhaltige Rasierhilfszubereitung nach einem der vorangehenden Ansprüche vorliegend als O/W-Emulsion **dadurch gekennzeichnet, daß** die Emulgatoren gewählt werden aus der Gruppe der Mono-, Di- und Trialkylphosphosäureester und deren Ethoxylate besonders bevorzugt Triceteareth-4 Phopsphat.

9. Emulgatorfreie Rasierhilfszubereitung nach einem der vorangehenden Ansprüche vorliegend als Gel **dadurch gekennzeichnet, daß** zusätzlich ein Gehalt an PEG-40-hydriertem Rizinusfettsäureether von 0,75 bis 2 Gew.% vorliegt.

10. Lipidfreie Rasierhilfszubereitung nach einem der vorangehenden Ansprüche **dadurch gekennzeichnet, daß** zusätzlich ein Gehalt an Zellulosederivaten, bevorzugt Hydroxyethylcellulose in Konzentrationen von 0,1 bis 1 Gew.%, bevorzugt 0,1 bis 0,6 Gew.%, besonders bevorzugt 0,2 Gew.%, vorliegt.

11. Verwendung von Rasierhilfszubereitungen nach einem der vorangehenden Ansprüche als Hilfsmittel bei der Elektrorasur.

12. Rasierapparat enthaltend Rasierhilfszubereitungen nach einem der vorangehenden Ansprüche.
